# EUROPÄISCHE PATENTSCHRIFT

(11) **EP 1 871 259 B1**
(45) Veröffentlichungstag und Bekanntmachung des Hinweises auf die Patenterteilung: **23.06.2010**
(21) Anmeldenummer: 06723605.9
(22) Anmeldetag: 21.03.2006
(51) Int. Cl.: A61B 18/14

(54) **ELEKTROCHIRURGISCHES INSTRUMENT**
ELECTROSURGICAL INSTRUMENT
INSTRUMENT ELECTRO-CHIRURGICAL

(30) Priorität: 24.03.2005 DE 102005013847
(43) Veröffentlichungstag der Anmeldung: 02.01.2008
(73) Patentinhaber: Erbe Elektromedizin GmbH, 72072 Tübingen (DE)
(72) Erfinder: HAFNER, Dieter, 72072 Tübingen (DE)
(74) Vertreter: Bohnenberger, Johannes
(86) Internationale Anmeldenummer: PCT/EP2006/002605
(87) Internationale Veröffentlichungsnummer: WO 2006/100046

(56) Entgegenhaltungen:
- US-A- 5 078 717
- US-A- 5 354 296
- US-A1- 2002 049 439

## Beschreibung

Die Erfindung betrifft ein elektrochirurgisches Instrument nach dem Oberbegriff des Patentanspruches 1.

Elektrochirurgische Instrumente werden seit vielen Jahren in der Hochfrequenz-Chirurgie eingesetzt, um biologisches Gewebe zu koagulieren oder zu schneiden. Bei einer Koagulation wird ein hochfrequenter Strom durch das zu behandelnde Gewebe geleitet, so dass sich dieses aufgrund Eiweißkoagulation und Dehydratation verändert. Das Gewebe zieht sich dabei derart zusammen, dass die Gefäße verschlossen und Blutungen gestillt werden. Für einen Schneidprozess ist eine hohe Stromdichte erforderlich, so dass durch ein explosionsartiges Verdampfen der Gewebeflüssigkeit und einem damit verbundenen Aufreißen der Zellmembranen das Gewebe vollständig durchtrennt wird.

Für die thermische Behandlung biologischen Gewebes werden sowohl monopolare, als auch bipolare Techniken angewendet.

Bei der monopolaren Technik wird der von einem HF-Generator an das elektrochirurgische Instrument zugeführte HF-Strom in das zu behandelnde Gewebe über eine differente Elektrode appliziert, wobei der Strompfad durch den Körper eines Patienten bis zu einer indifferenten Neutralelektrode führt und von dort zurück zum HF-Generator. An der differenten Elektrode ist eine hohe Stromdichte pro Flächeneinheit zur Behandlung vorgesehen, während bei der indifferenten Elektrode die Stromdichte pro Flächeneinheit gegenüber der differenten Elektrode deutlich verringert ist. Dies lässt sich durch ein entsprechend großflächiges Auslegen der Neutralelektrode erreichen. Das monopolare Schneiden eignet sich insbesondere für große Schnitte, beispielsweise, um Fettgewebe zu durchtrennen.

Die bipolare Technik wird dann eingesetzt, wenn der HF-Strom mit hoher Präzision appliziert werden soll. Bipolare Instrumente weisen meist zwei gelenkig miteinander verbundene Klemmenteile auf, wobei an deren proximalen Enden Griffeinrichtungen zur Handhabung der Klemmenteile vorgesehen sind. An distalen Enden der Klemmenteile befinden sich Elektrodenteile zum Durchleiten des HF-Stromes von dem HF-Generator durch das zu behandelnde Gewebe. Hierfür sind die Elektrodenteile über Stromzuführungseinrichtungen an den HF-Generator anschließbar. Meist sind die Elektrodenteile derart ausgelegt, dass sie auch zum Fassen und Einklemmen des zu behandelnden Gewebes geeignet sind. Aufgrund des kurzen Stromweges zwischen den Elektrodenteilen des bipolaren Instruments lässt sich der Stromweg besser kalkulieren, als dies bei monopolaren Anordnungen der Fall ist, da er nicht weite Strecken durch den Körper des Patienten verläuft.

Ein Gerät nach dem allgemeinen Teil von Anspruch 1 ist aus US 5 078 717 bekannt.

Aus der WO 99/37228 ist ein bipolares Koagulations- und Schneidgerät für die endoskopische Chirurgie bekannt. Es sind zwei Branchen vorgesehen, die über ein axial verschiebbares Rohrteil bewegbar sind. Die Branchen weisen an distalen Enden einander gegenüberliegende, flächige, im Inneren jeweils mit einem Lochbereich ausgebildete Koagulationselektroden auf, um ein zu behandelndes Gewebe mit diesen zu fassen und zu koagulieren. Ferner ist eine stangenförmig ausgebildete Schneidelektrode vorgesehen, die ebenfalls über das Rohrteil bewegbar ist und die zwischen den Koagulationselektroden durch den Lochbereich an dem eingeklemmten Gewebe angreift. Durch eine sukzessive lineare Verschiebung des Rohrteiles setzt die Schneidelektrode - ähnlich dem Schnitt einer Schere - immer weiter auf dem zu behandelnden Gewebe auf, bis sie die gewünschte Schnittlinie vollständig überdeckt hat.

Der Schneidbereich der Schneidelektrode eines elektrochirurgischen Instruments ist sowohl bei der monopolaren als auch bei der bipolaren Technik, wie mit der WO 99/37228 gezeigt, beständig hohen Belastungen ausgesetzt, da eine für den Schneidvorgang an der Elektrode erforderliche hohe Stromdichte den Verschleiß der Elektrode fördert.

Andere elektrochirurgische Instrumente sehen äußerst klein konzipierte Schneidelektroden vor (z. B. Nadelelektrode), so dass eine gleichzeitige Koagulation aufgrund kleiner Koagulationsflächen nicht hinreichend durchgeführt werden kann. Gerade bei Hohlorganen, wie z. B. Blutgefäßen, lässt sich so eine gezielte Thermofusion kaum durchführen.

Oftmals werden elektrochirurgische Instrumente mit einer mechanisch zu betätigenden Schneide ausgebildet. Nach einer erfolgten Koagulation kann das behandelte Gewebe mittels der Schneide vollständig durchtrennt werden. Die dabei von dem Chirurgen beim Schneiden aufzubringende Kraft bewirkt ebenfalls einen hohen Verschleiß der Schneide, so dass bereits nach kurzer Zeit die Qualität des Schnittes erheblich sinkt. Insofern muss die Schneidelektrode häufig ausgewechselt werden, was aufgrund einer relativ komplexen Mechanik oftmals schwierig ist. Gegebenenfalls ist sogar das gesamte elektrochirurgische Instrument nicht mehr zu verwenden und muss daher komplett ausgewechselt werden.

Der Erfindung liegt daher die Aufgabe zu Grunde, ein elektrochirurgisches Instrument der eingangs genannten Art dahin gehend weiterzubilden, dass der Schneidvorgang bei gleichzeitiger Standzeitverlängerung der Schneidelektrode mit erhöhter Zuverlässigkeit durchführbar ist.

Diese Aufgabe wird durch ein elektrochirurgisches Instrument nach Patentanspruch 1 gelöst.

Ein wesentlicher Punkt der Erfindung liegt darin, dass aufgrund der Ausgestaltung der Schneidelektrode ein großer Schneidbereich zur Verfügung steht, wobei der Schneidbereich aufgrund der Rotation der Schneidelektrode jeweils nur abschnittsweise im Einsatz ist. Das heißt, dass nur zwischen einzelnen Abschnitten des Schneidbereiches und dem zu behandelnden Gewebe ein Lichtbogen erzeugbar und so ein sukzessiver Schneidevorgang durchführbar ist. Damit lässt sich ein etwaiger Verschleiß auf die einzelnen Abschnitte verteilen, so dass sich die Standzeit der Schneidelektrode erhöht. Beim Schneiden mit hochfrequentem Wechselstrom ist ein Schnitt durch das Gewebe nur möglich, wenn die elektrische Spannung zwischen der aktiven Elektrode und dem Gewebe so groß ist, dass elektrische Lichtbogen zünden. Läuft nun der Schneidbereich über das Gewebe, zünden die Lichtbogen überall dort, wo der Abstand zwischen dem Schneidbereich und dem Gewebe ausreichend klein ist. Der Bereich des Schneidbereiches, der die Zündung der Lichtbogen ermöglicht ist demnach der wirksame Abschnitt des Schneidbereiches.

Aufgrund der Ausgestaltung der Schneidelektrode mit dem wirksamen Abschnitt wird die Erzeugung eines linearen Schnittes an dem zu behandelnden Gewebe über die Rotation der Schneidelektrode ermöglicht. Zur Ausführung der linearen Schnittbewegung wird letztendlich die Rotationsbewegung genutzt und somit bei hinreichend hoher Windungszahl pro Längeneinheit eine Untersetzung (ins Langsame) erzeugt. Es lassen sich also besonders präzise Schnitte erzielen, was vor allem bei sehr kurzen Schnittlängen vorteilhaft ist. Da die Raumkurve zur Ausbildung des Schneidbereiches letztendlich durch eine bestimmte Anzahl von Windungen definiert ist, lässt sich zudem die Schnittgeschwindigkeit, d. h: die Geschwindigkeit, mit der sich die wirksamen Abschnitte über das Gewebe bewegen, anhand der Anzahl der Windungen einstellen. Die Schnittgeschwindigkeit der wirksamen Abschnitte verhält sich umgekehrt proportional zur Anzahl der Windungen pro Längeneinheit bei gleich bleibender Rotationsgeschwindigkeit. Das Fortschreiten der Einzel-Lichtbögen über die Gesamtschnittlänge wird dabei auch aufgrund der Schrauben-Untersetzungswirkung langsamer.

Eine erste bevorzugten Ausführungsform sieht vor, dass die Schneidelektrode ein schraubenlinienförmig ausgebildetes Stabelement umfasst, der Schneidbereich als Schraubenkurve auf dem Stabelement ausgebildet ist und der Abschnitt des Schneidbereiches entlang der Schraubenkurve an dem Stabelement bei Drehung der Schneidelektrode entlang läuft. Die Schraubenkurve für den Schneidbereich ist auf diese Weise besonders einfach herstellbar. Dabei kann das Stabelement derart gewickelt sein, dass der Abschnitt entlang einer Fläche des mindestens einen Schneidbereiches verläuft oder aber entlang einer Kante. Verläuft der Abschnitt entlang der Kante, so ist an diesem eine höhere Stromdichte erzielbar.

Eine weitere bevorzugten Ausführungsform sieht vor, dass die Schneidelektrode ein tordiertes Flächenelement umfasst, an dessen Rändern zwei Schneidbereiche als Schraubenkurven ausgebildet sind und der Abschnitt der Schneidbereiche entlang der Schraubenkurven an dem Flächenelement bei Drehung der Schneidelektrode entlang läuft. Damit ist auf äußerst einfache Weise ein schraubenlinienförmig verlaufender Schneidbereich ausbildbar. Zudem ist die so ausgebildete Schneidelektrode äußerst stabil und widersteht mechanischen Beanspruchungen in hohem Maße.

Auch lässt sich das oben beschriebene Stabelement derart tordieren und anordnen, so dass letztendlich vier Schneidbereiche als Schraubenkurven ausgebildet sind und der jeweilige wirksame Abschnitt der Schneidbereiche entlang der Schraubenkurven an dem Stabelement bei Drehung der Scheidelektrode entlang läuft.

Die Schneidelektrode kann im Prinzip als beliebige Raumkurve ausgebildet sein, so dass die Raumkurve beispielsweise eine Umhüllende eines Kegels oder dergleichen geometrische Figur beschreibt.

Vorzugsweise ist die Schneidelektrode mit mindestens einer Windung, vorzugsweise mit genau einer Windung ausgebildet. Damit ist exakt jeweils ein wirksamer Abschnitt des Schneidbereiches mit dem Gewebe in Wechselwirkung und der Schnitt lässt sich präzise und nachvollziehbar ausführen.

Die Schneidelektrode lässt sich alternativ auch als zylinderförmiger Hohlkörper oder als Stangenvollmaterial ausbilden, auf dem der schraubenlinienförmige Schneidbereich angeordnet ist. Dabei kann der Hohlkörper oder das Vollmaterial selbst elektrisch isolierend vorgesehen sein. Sind der Hohlkörper oder das Vollmaterial aus elektrisch leitendem Material mit einer an der Mantelfläche vorgesehenen Isolierschicht ausgebildet, so kann der schraubenlinienförmige Schneidbereich durch teilweises entfernen der Isolierschicht, z. B. durch Fräsen oder über einen mittels Lichtbogen durchgeführten Schneidprozess, freigelegt werden. Eine derart ausgebildete Schneidelektrode ist äußerst stabil und verschleißresistent, insbesondere bei Verwendung eines Vollmaterials.

Vorzugsweise sind zwei gelenkig miteinander verbundene Branchen vorgesehen, die entsprechend einem Klemm- oder Schneidwerkzeug betätigbar sind und somit eine Halteeinrichtung mit jeweils mindestens einem Fasselement an den Branchen zum Fassen des zu behandelnden Gewebes ausbilden. Das Einspannen des Gewebes bedingt dessen Fixierung, so dass auf diese Weise ein präziser Schnitt erreicht werden kann. Da das Gewebe zwischen den Fasselementen eingeklemmt ist, muss die Halteeinrichtung derart ausgebildet sein, dass die Schneidelektrode ungehindert mit dem zu behandelnden, eingeklemmten Gewebe wechselwirken kann. Das Schneiden mittels des HF-Stromes erfolgt im Wesentlichen berührungslos über Schneidfunken, d. h. mittels Lichtbogen, so dass die Schneidelektrode nur um sich selbst drehbar angeordnet sein muss und im Übrigen ortsfest zu lagern ist. Mindestens eines der Fasselemente weist daher einen Aufnahme-Durchlass-Bereich für die Schneidelektrode auf, wobei ein Aufnahmebereich für die Lagerung der Schneidelektrode in oder an den Fasselementen vorgesehen ist, während ein Durchlassbereich die Zugänglichkeit des Schneidfunkens an das zu behandelnde Gewebe ermöglicht. Das heißt, der Lichtbogen ist über den Durchlassbereich zwischen der Schneidelektrode und dem in der Halteeinrichtung eingeklemmten Gewebe erzeugbar. Wird nun die Schneidelektrode um die Rotationsachse gedreht, wandert der wirksame Abschnitt des Schneidbereiches entlang des Durchlassbereiches und das eingeklemmte Gewebe wird durchtrennt.

Vorteilhafterweise ist der Aufnahme-Durchlass-Bereich derart ausgebildet, dass er den definierten Fensterbereich aufweist. Sind beispielsweise der HF-Schneidstrom oder die Spannung derart geregelt, dass Abschnitte des Schneidbereiches an dem Gewebe wirksam werden, wenn sie einen definierten Mindestabstand zu dem Gewebe unterschreiten, so ist der Aufnahme-Durchlass-Bereich dergestalt auszulegen, dass eine Behinderung der Schneidelektrode durch die Halteeinrichtung verhindert wird. Gleichzeitig muss eine ausreichende Fixierung des Gewebes gewährleistet sein. An die Ausgestaltung des Aufnahme-Durchlass-Bereiches sind in diesem Falle keine weiteren Anforderungen zu stellen. Ist die Wirksamkeit des wirksamen Abschnittes des Schneidbereiches jedoch von der Ausgestaltung, insbesondere von der Größe des Aufnahme-Durchlass-Bereiches abhängig, so ist dieser vorzugsweise als der definierte Fensterbereich ausgebildet. Der definierte Fensterbereich legt ausschließlich den Gewebebereich frei, an dem der Schnitt auszuführen ist. Das übrige Gewebe wird durch das den Fensterbereich festlegende Fasselement abgedeckt, so dass an diesem eine Stromeinwirkung nicht stattfinden kann. Bei Rotation der Schneidelektrode wandert der wirksame Abschnitt entlang des Fensterbereichs. Der definierte Fensterbereich ermöglicht eine äußerst präzise Schnittführung, weil der Schnittbereich exakt abgegrenzt ist. Zudem ist die Ausführung des Schnittes von sonstigen Parametern, wie z. B. eingestellte Stromstärke bzw. Spannung in Abhängigkeit vom Abstand des Schneidbereiches zu dem zu behandelnden Gewebe, (natürlich in bestimmten Grenzen) weitestgehend unabhängig.

In der praktischen Anwendung weist die Halteeinrichtung eine erste Elektrode und eine zweite Elektrode zum Durchleiten eines Koagulationsstromes für den Koagulationsvorgang durch das Gewebe als Fasselemente an jeweils den Branchen auf, so dass das elektrochirurgische Instrument in erster Linie als bipolare Anordnung vorgesehen ist. Vor dem eigentlichen Schneidprozess lässt sich das eingeklemmte Gewebe, z. B. ein Gefäß, somit zuerst koagulieren und anschließend mittels der Schneidelektrode durchtrennen. Die Schneidelektrode kann dann mit einer der Koagulationselektroden während des Schneidvorganges zusammenwirken, so dass Schneid- und entsprechende Koagulationselekttode wiederum eine bipolare Anordnung ausbilden. Auch ein Zusammenwirken der Schneidelektrode mit der oben bereits beschriebenen Neutralelektrode ist möglich.

Vorzugsweise weist der Aufnahme-Durchlass-Bereich mindestens an in Richtung der Schneidelektrode weisenden Flächenbereichen eine Isolationsschicht auf, so dass die Elektrode, die der Elektrode mit dem Aufnahme-Durchlass-Bereich gegenüberliegt und die Schneidelektrode während des Schneidvorganges eine bipolare Anordnung ausbilden.

Damit ist sichergestellt, dass während des Schneidvorganges keine Wechselwirkung zwischen der den Aufnahme-Durchlass-Bereich aufweisenden Elektrode und der Schneidelektrode auftreten.

Weist die Halteeinrichtung zwei Koagulationselektroden auf, so sind die erste Elektrode, die zweite Elektrode und die Schneidelektrode z. B. derart zueinander angeordnet, dass die zweite Elektrode zwischen der ersten Elektrode und der Schneidelektrode ausgebildet ist. Das heißt, die Schneidelektrode erstreckt sich in diesem Falle unterhalb bzw. außerhalb der Halteinrichtung. Das zu behandelnde Gewebe ist also über die erste und die zweite Elektrode einklemmbar und kann in einem ersten Schritt koaguliert werden. Während des Koagulationsvorganges ist die Schneidelektrode inaktiv. In einem zweiten Schritt wird mittels der Schneidelektrode an dem koagulierten Gewebe ein Schneidvorgang ausgeführt. Um den Zugang für die Schneidelektrode zu ermöglichen, weist mindestens die zweite Elektrode den Aufnahme-Durchlass-Bereich auf. Die Flächenbereiche des Aufnahme-Durchlass-Bereiches, die in Richtung der Schneidelektrode ausgebildet sind, weisen zudem die Isolationsschicht auf. Damit wird eine Wechselwirkung zwischen der Schneidelektrode und der zweiten Elektrode, beispielsweise durch unkontrollierte Funkenbildung, vermieden. Die erste Elektrode und die Schneidelektrode bilden während des Schneidvorganges eine bipolare Anordnung aus. Vorzugsweise ist auch bei dieser Ausführungsform der Aufnahme-Durchlass-Bereich als definierter Fensterbereich ausgebildet.

Selbstverständlich kann die Schneidelektrode auch überhalb der Halteinrichtung angeordnet sein, so dass die erste Elektrode den Aufnahme-Durchlass-Bereich bzw. den Fensterbereich aufweist.

Alternativ ist es möglich, dass die Schneidelektrode innerhalb einer der Koagulationselektroden gelagert ist. Dazu sind die erste Elektrode, die zweite Elektrode und die Schneidelektrode derart zueinander angeordnet, dass die Schneidelektrode zwischen der ersten Elektrode und der zweiten Elektrode ausgebildet ist. Die Schneidelektrode ist in dieser Ausführungsform also zwischen den Koagulationselektroden (bzw. zwischen den Fasselementen) untergebracht und somit beispielsweise vor mechanischer Beanspruchung geschützt. Dazu weist entweder die erste Elektrode oder die zweite Elektroden den Aufnahme-Durchlass-Bereich auf, wobei der Aufnahme-Durchlass-Bereich auch hier an den Flächenbereichen in Richtung der Schneidelektrode mit der Isolationsschicht vorgesehen ist, so dass die dem Aufnahme-Durchlass-Bereich gegenüberliegende Elektrode und die Schneidelektrode während des Schneidvorganges die bipolare Anordnung ausbilden. Vorzugsweise ist der Aufnahme-Durchlass-Bereich derart ausgebildet, dass er als der definierte Fensterbereich vorgesehen ist. Damit ist eine präzise Schnittführung gewährleistet.

In einer weiteren bevorzugten Ausführungsform weist die Halteeinrichtung eine Elektrode als ein Fasselement und ein elektrisch isolierendes weiteres Fasselement auf, wobei die Elektrode und die Schneidelektrode derart zueinander angeordnet sind, dass sie während des Schneidvorganges eine bipolare Anordnung ausbilden. Auch bei dieser Ausführungsform lassen sich alle oben bereits beschriebenen Varianten verwirklichen. Das heißt, die Schneidelektrode kann zwischen den Fasselementen oder auch über- bzw. unterhalb derselben angeordnet sein. Ist ein Fasselement als elektrisch isolierendes Element ausgebildet, muss keine explizite Isolationsschicht zwischen diesem Element und der Schneidelektrode vorgesehen werden. Damit lässt sich der Aufnahme-Durchlass-Bereich bzw. der Fensterbereich für die Schneidelektrode besonders einfach ausbilden.

Durch Variation der Ausmaße des Durchlassbereiches, insbesondere des definierten Fensterbereiches, können unterschiedliche Behandlungsmöglichkeiten realisiert werden. So wird durch einen schmaleren Fensterbereich, insbesondere bei Ausgestaltung der Fasselemente als Koagulationselektroden, eine Koagulationsfläche verbreitert. Damit ist eine optimale Koagulation des zu behandelnden Gewebes gewährleistet. Umgekehrt kann im zu behandelnden Gewebe während des Koagulationsvorganges mit einem breiteren Fensterbereich und einem breiteren Durchlassbereich Feuchtigkeit erhalten werden, so dass sich eine notwendige Schnittenergie reduzieren lässt.

Alternativ ist es möglich, die Halteeinrichtung durch zwei elektrisch isolierende Fasselemente auszubilden. Die Halteeinrichtung dient dann lediglich dem Einklemmen und Fixieren des zu behandelnden Gewebes, während eine Gegenelektrode zur Schneidelektrode beispielsweise als eine am Patienten befestigte Neutralelektrode ausgebildet ist.

Eine erfindungsgemäße Lösung sieht vor, dass das dem Aufnahme-Durchlass-Bereich gegenüberliegende Fasselement einen hervorstehenden Bereich in Richtung Aufnahme-Durchlass-Bereich zur Heranführung des zu behandelnden Gewebes an und/oder zur Einführung des zu behandelnden Gewebes in den Aufnahme-Durchlass-Bereich aufweist. Mit dieser Maßnahme lässt sich die Schnittführung präzisieren, beispielsweise durch verbesserte Führung des Schneidfunkens. Zudem bleibt eine Feuchtigkeit des Gewebes im Durchlassbereich länger erhalten, so dass sich die notwendige Schnittenergie reduzieren lässt.

Eine bevorzugte Ausführungsform sieht vor, dass die Fasselemente jeweils mindestens einen Spannbereich aufweisen, derart, dass beim Einklemmen des Gewebes dieses zwischen den Fasselementen vorgespannt ist und der Schneidvorgang mittels der Schneidelektrode an dem vorgespannten Gewebe durchführbar ist. Das Gewebe wird durch die Spannbereiche beidseitig in Richtung deren Endbereiche gezogen, d. h. gestrafft. Das unter Spannung stehende Gewebe lässt sich präziser und sauberer durchtrennen, weil sich Fasern des Gewebes quer zu einer Schneidrichtung ausrichten und das Gewebe dabei dünner wird.

Der Spanneffekt kann durch ein Oberflächenprofil an den Fasselemente weiter verstärkt werden. Dazu ist das Profil vorzugsweise an Endbereichen mindestens an einem Spannbereich ausgebildet und bewegt das Gewebe zusätzlich in einer durch die Spannbereiche definierten Zugrichtung bzw. verhindert ein Zurückweichen des Gewebes entgegen dieser Zugrichtung.

Vorzugsweise ist das den Spanneffekt unterstützende Oberflächenprofil als Sägezahnprofil ausgebildet. Zähne des Profils können beispielsweise so angeordnet sein, dass sie während des Zusammenführens der Branchen immer weiter in das Gewebe greifen und dieses in Zugrichtung mitnehmen. Damit wird die Spannung im Gewebe deutlich erhöht. Es ist allerdings darauf zu achten, dass durch das Profil eine Versetzung des Gewebes vermieden wird, so dass die Zähne vorzugsweise als abgerundete Noppen ausgebildet sind.

Vorzugsweise ist einer der Spannbereiche mindestens in einem ersten mittleren Abschnitt konvex, der ihm gegenüberliegende Spannbereich mindestens in einem zweiten mittleren Abschnitt konkav gekrümmt. Damit passen die Spannbereiche bei einem Zusammenführen der Branchen im Wesentlichen formschlüssig ineinander. Durch die gekrümmten Spannbereiche wird auf einfachste Art ein Spannen des Gewebes ermöglicht, weil dieses durch die gekrümmten Bereiche gestreckt wird. Durch den Formschluss ist das Gewebe dann zwischen den Branchen in gespanntem Zustand sicher arretiert.

Um die Rotation der Schneidelektrode zu ermöglichen, ist dem elektrochirurgischen Instrument ein Rotationsantrieb zugeordnet. Die Rotation wird damit selbsttätig durchgeführt, ohne dass ein Operateur hierauf achten müsste, sofern es sich z. B. um einen elektrischen Antrieb handelt. Auch ein mechanischer Antrieb ist denkbar. Der Antrieb ist dann z. B. derart ausgebildet, dass er über ein am elektrochirurgischen Instrument ausgebildetes Fingerrad betätigbar ist.

Weitere Ausführungsformen der Erfindung ergeben sich aus den Unteransprüchen.

Nachfolgend wird die Erfindung anhand von Ausführungsbeispielen beschrieben, die anhand der Abbildungen näher erläutert werden. Hierbei zeigen
- - Fig. 1: ein perspektivisch dargestelltes elektrochirurgisches Instrument mit einer erfindungsgemäßen Fasselement- und/oder Elektrodenanordnung in einer ersten bevorzugten Ausführungsform;
- - Fig. 2: eine perspektivisch dargestellte Fasselement- und/oder Elektrodenanord- nung gemäß der ersten bevorzugten Ausführungsform;
- - Fig. 3: eine schematisch im Schnitt dargestellte Fasselement- und/oder Elektro- denanordnung in Vorderansicht gemäß der ersten bevorzugten Ausfüh- rungsform;
- - Fig. 4: eine perspektivisch dargestellte Fasselement- und/oder Elektrodenanord- nung gemäß einer zweiten bevorzugten Ausführungsform;
- - Fig. 5: eine perspektivisch dargestellte Fasselement- und/oder Elektrodenanord- nung gemäß einer dritten bevorzugten Ausführungsform;
- - Fig. 6: eine perspektivisch dargestellte Schneidelektrode;
- - Fig. 7: eine perspektivisch dargestellte Schneidelektrode;
- - Fig. 8: eine schematisch im Schnitt dargestellte Fasselement- und/oder Elektro- denanordnung in Vorderansicht gemäß einer vierten bevorzugten Ausfüh- rungsform;
- - Fig. 9: eine schematisch im Schnitt dargestellte Fasselement- und/oder Elektro- denanordnung in Vorderansicht gemäß einer fünften bevorzugten Ausfüh- rungsform;
- - Fig. 10: eine schematisch im Schnitt dargestellte Fasselement- und/oder Elektro- denanordnung in Vorderansicht gemäß einer sechsten bevorzugten Ausfüh- rungsform;
- - Fig. 11: eine schematisch im Schnitt dargestellte Fasselement- und/oder Elektro- denanordnung in Vorderansicht gemäß einer siebten bevorzugten Ausfüh- rungsform;
- - Fig. 12: eine perspektivisch dargestellte Elektrodenanordnung gemäß einer achten bevorzugten Ausführungsform.

In der nachfolgenden Beschreibung werden für gleiche und gleich wirkende Teile dieselben Bezugsziffern verwendet.

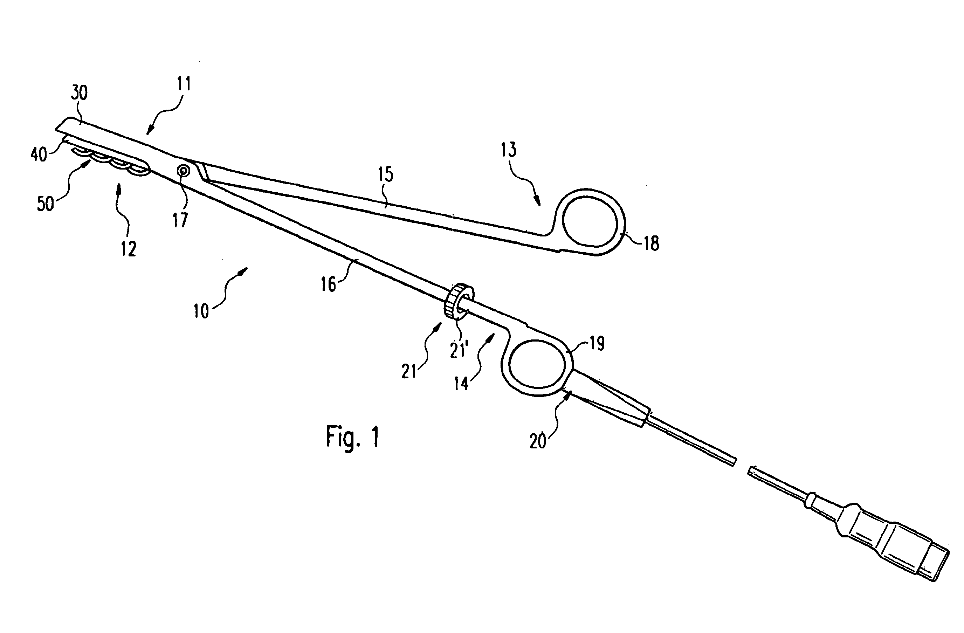

Fig. 1 zeigt ein perspektivisch dargestelltes elektrochirurgisches Instrument 10 mit einer erfindungsgemäßen Fasselement- und/oder Elektrodenanordnung in einer ersten bevorzugten Ausführungsform. Das Instrument 10 ist für einen Eingriff am eröffneten Körper ausgebildet und zur Behandlung von biologischem Gewebe 80 vorgesehen. In der Abbildung sind mit den Bezugsziffern 15 und 16 zwei Branchen des elektrochirurgischen Instruments 10 bezeichnet. Die beiden Branchen 15, 16 sind über eine Achse 17 miteinander verbunden und um diese verschwenkbar. Sie weisen mit einem ersten Fasselement 30 und einem zweiten Fasselement 40 versehene distale Enden 11, 12 auf, wobei sich die Fasselemente 30, 40 einander gegenüberliegen und zum Fassen und Fixierten des zu behandelnden Gewebes vorgesehen sind. Weiterhin ist eine schraubenlinienförmig ausgebildete Schneidelektrode 50 vorgesehen, die in diesem Falle unterhalb der beiden Fasselemente 30, 40 angeordnet ist. Die Schneidelektrode 50 ist um eine Rotationsachse drehbar, so dass dem elektrochirurgischen Instrument 10 vorzugsweise ein Rotationsantrieb 21 zugeordnet ist. Der Rotationsantrieb 21 ist in diesem Falle über ein Fingerrad 21' betätigbar, so dass der Operateur die Schneidelektrode 50 beispielsweise manuell betätigen, d. h. drehen kann. Aufgrund dieser Ausgestaltung der Schneidelektrode 50 steht ein großer, entlang der Schraubenlinie ausgebildeter Schneidbereich 51 zur Verfügung, wobei der Schneidbereich 51 aufgrund der Rotation der Schneidelektrode 50 jeweils nur abschnittsweise im Einsatz ist. Damit lässt sich ein etwaiger Verschleiß auf die einzelnen Abschnitte des Schneidbereiches verteilen, so dass sich die Standzeit der Schneidelektrode 50 erhöht. Das heißt, durch das Wandern des Belastungspunktes wird die Lebensdauer im Vergleich zu herkömmlichen Elektroden (z. B. Nadelelektrode) erhöht.

Zur Ausführung der linearen Schnittbewegung wird letztendlich die Rotationsbewegung genutzt und somit bei hinreichend hoher Windungszahl pro Längeneinheit eine Untersetzung (ins Langsame) erzeugt. Es lassen sich also besonders präzise Schnitte erzielen, was vor allem bei sehr kurzen Schnittlängen vorteilhaft ist. Da die Raumkurve zur Ausbildung des Schneidbereiches letztendlich durch eine bestimmte Anzahl von Windungen definiert ist, lässt sich zudem die Schnittgeschwindigkeit, d. h. die Geschwindigkeit, mit der sich die wirksamen Abschnitte über das Gewebe bewegen, anhand der Anzahl der Windungen einstellen. Die Schnittgeschwindigkeit der wirksamen Abschnitte verhält sich umgekehrt proportional zur Anzahl der Windungen pro Längeneinheit bei gleich bleibender Rotationsgeschwindigkeit. Das Fortschreiten der Einzel-Lichtbögen über die Gesaintschnittlänge wird dabei aufgrund der Schrauben-Untersetzungswirkung langsamer. Zur näheren Ausführung der Schneidelektrode 50 sei auf nachfolgende Figurenbeschreibung verwiesen.

Weiterhin sind Griffteile 18, 19 an jeweiligen proximalen Enden 13, 14 des elektrochirurgischen Instruments 10 vorgesehen. Das proximale Ende 14 der Branche 16 endet in einem Stromanschlusselement bzw. einer Stromzuführungseinrichtung 20 zum Anschließen des elektrochirurgischen Instruments 10 an einen (nicht dargestellten) HF-Generator, der eine HF-Spannung erzeugt, so dass ein HF-Strom beispielsweise durch in dem Instrument 10 laufende elektrische Leitungen (nicht gezeigt) mindestens der Schneidelektrode 50 zugeführt werden kann. Alternativ ist es möglich, an beiden Branchen 15, 16 eine Stromzuführung bereitzustellen.

Die Fasselemente 30, 40 können bei dieser Ausführungsform als Koagulationselektroden ausgebildet sein, so dass sich eine erste Koagulationselektrode und eine zweite Koagulationselektrode gegenüberliegen. Damit wird vor dem Durchtrennen des fixierten Gewebes dessen Koagulation ermöglicht, indem die Koagulationselektroden während des Koagulationsvorganges eine bipolare Anordnung ausbilden. Da die Schneidelektrode 50 dann als dritte Elektrode an dem elektrochirurgischen Instrument 10 angebracht ist, müssen die Fasselemente, hier also die Koagulationselektroden derart ausgebildet sein, dass sich auch während des Schneidvorganges eine bipolare Anordnung mit der Schneidelektrode realisieren lässt.

Prinzipiell sind die Fasselemente 30, 40 auch als elektrisch isolierende Elemente ausbildbar und würden somit lediglich der Fixierung des zu behandelnden Gewebes dienen. Dann würde die Schneidelektrode 50 als differente Elektrode zusammen mit einer am Patienten anzubringenden indifferenten Neutralelektrode (nicht gezeigt) eine monopolare Anordnung ausbilden. Zur genauen Ausgestaltung derartiger Anordnungen sei auf nachfolgende Figurenbeschreibung verwiesen.

Das in Fig. 1 gezeigte elektrochirurgische Instrument 10 ist, wie bereits oben erwähnt, für den Einsatz am eröffneten Körper ausgebildet. Das Prinzip der erfindungsgemäßen Schneidelektrode 50 ist ebenso für die Endoskopie, z. B. bei laparoskopischen Instrumenten anzuwenden. Die an den Branchen 15, 16 befestigten Fasselemente 30, 40 und die Schneidelektrode 50 sind dann beispielsweise über einen an einem Schaft befestigten Handgriff zu betätigen oder aber es ist eine Steuerungseinheit vorgesehen, so dass eine Betätigung der Fasselemente und der Schneidelektrode über diese gesteuert wird.

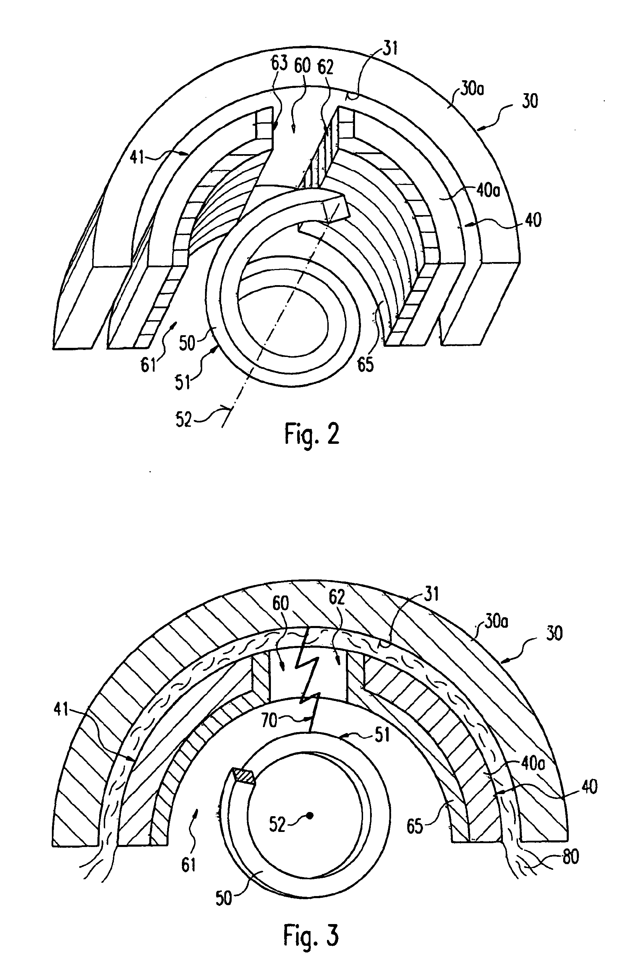

Fig. 2 zeigt eine stark vergrößerte, perspektivisch dargestellte Fasselement- und/oder Elektrodenanordnung gemäß der ersten bevorzugten Ausführungsform, wie sie auch aus Fig. 1 zu entnehmen ist. Die Fasselemente 30, 40 sind in diesem Ausrührungsbeispiel als Koagulationselektroden, d. h. als erste Koagulationselektrode 30a und als zweite Koagulationselektrode 40a ausgebildet, derart, dass sich die erste Elektrode 30a bei Zusammenführung der Branchen 15, 16 über die zweite Elektrode 40a stülpt, d. h., diese abdeckt. Wie aus der Abbildung ersichtlich, sind die Elektroden 30a, 40a gekrümmt ausgebildet. Dabei weist die zweite Elektrode 40a eine konvexe Krümmung auf und die erste Elektrode 30a, die der konvex ausgebildeten Elektrode 40a gegenüberliegt, weist eine konkave Krümmung auf. Auf diese Weise passen die Elektroden 30a, 40a bei zusammengeführten Branchen 15, 16 im Wesentlichen formschlüssig ineinander. Durch die gekrümmten Elektroden 30a, 40a wird das Gewebe in Richtung von Endbereichen der Elektroden 30a, 40a gezogen, d. h. gestreckt bzw. gestrafft. Die Elektroden 30a, 40a bilden demgemäß Spannbereiche 31, 41 aus. Damit lässt sich das Gewebe präziser schneiden, da sich Fasern des Gewebes quer zu einer Schneidrichtung ausrichten und das Gewebe dabei dünner wird. Durch den Formschluss ist das Gewebe dann zwischen den Elektrode 30a, 40a in gespanntem Zustand fixiert. In diesem Ausführungsbeispiel sind die Elektroden 30a, 40a im Wesentlichen vollständig als Spannbereiche 31, 41 ausgebildet. Alternativ ist es möglich, dass nur Abschnitte der Elektroden Spannbereiche ausbilden.

Alternativ können die Spannbereiche mit unterschiedlichen Krümmungsradien ausgebildet sein; der Krümmungsradius des konkav ausgebildeten Spannbereichs ist beispielsweise größer als der Krümmungsradius des konvex ausgebildeten. Die Krümmungen verlaufen dann um Längsachsen der distalen Enden derart, dass das zwischen den distalen Enden gehaltene und senkrecht zu den Längsachsen verlaufende Gewebe mit zu mittleren Abschnitten der Spannbereiche hin steigender Pressung gehalten wird. Vorteilhafterweise wird hier das einmal eingeklemmte Gewebe aufgrund einer steigenden Pressung besonders sicher zwischen den Spannbereichen arretiert. Ein Abgleiten des einmal erfassten Gewebes aus den Elektrodenteilen ist somit ausgeschlossen. Zudem wird an den Bereichen hoher Pressung, also hohen Drucks, bedingt durch die hohe Klemmkraft, ein sicheres Verschließen des Gewebes während des Koagulationsvorganges erreicht.

Die Schneidelektrode 50 ist gemäß Fig. 2 unterhalb der zweiten Elektrode 40a angeordnet, d. h. die zweite Elektrode 40a ist zwischen der ersten Elektrode 30a und der Schneidelektrode 50 vorgesehen. Aufgrund dieser Anordnung ist es notwendige, die Zugänglichkeit der Schneidelektrode 50 an dem zwischen den Fasselementen bzw. zwischen den Koagulationselektroden eingeklemmten Gewebe zu ermöglichen. In der praktischen Anwendung ist ein berührungsloses Schneiden mittels Schneidfunken vorgesehen, so dass die Schneidelektrode nur um sich selbst drehbar angeordnet und im Übrigen ortsfest gelagert ist. Die zweite Elektrode 40a weist daher einen Aufnahme-Durchlass-Bereich 60 auf, wobei ein Aufnahmebereich 61 für die Lagerung der Schneidelektrode 50 vorgesehen ist, während ein Durchlassbereich 62 die Zugänglichkeit des Schneidfunkens an das zu behandelnden Gewebe ermöglicht. Das heißt, die Schneidelektrode 50 rotiert zur Ausnutzung des gesamten Schneidbereiches 51 um die Rotationsachse 52, wobei abschnittsweise Schneidbereiche 51 derart nah an das eingeklemmte Gewebe herangeführt werden, dass ein Lichtbogen über den Durchlassbereich 62 zwischen der Schneidelektrode 50 und dem in der Halteeinrichtung (Fasselemente) eingeklemmten Gewebe erzeugbar ist.

Üblicherweise ist der Durchlassbereich 62 als ein definierter Fensterbereich 63 vorgesehen oder weist zumindest den definierten Fensterbereich 63 auf. Das heißt, der Durchlassbereich 62 ist derart ausgestaltet, dass er ausschließlich einen gewünschten Gewebebereich an dem eingeklemmten Gewebe freilegt. Damit wird eine unerwünschte Wechselwirkung zwischen Schneidelektrode 50 und Gewebe an nicht für den Schneidvorgang vorgesehenen Gewebestellen vermieden. Der definierte Fensterbereich 63 ermöglicht eine äußerst präzise Schnittführung, weil der Schnittbereich exakt abgegrenzt ist. Zudem ist die Ausführung des Schnittes von sonstigen Parametern, wie z. B. eingestellte Stromstärke bzw. Spannung in Abhängigkeit vom Abstand der Schneidbereiche 51 der Schneidelektrode 50 zu dem zu behandelnden Gewebe, weitestgehend unabhängig.

Sind jedoch beispielsweise der HF-Schneidstrom bzw. die Spannung derart geregelt, dass der wirksame Abschnitt des Schneidbereiches 51 an dem Gewebe wirksam wird, wenn er einen definierten Mindestabstand zu dem Gewebe unterschreitet, so ist der Aufnahme-Durchlass-Bereich 60 dahin gehend auszugestalten, dass eine Behinderung der Schneidelektrode 50 durch die Halteeinrichtung verhindert wird. Trotzdem muss eine ausreichende Fixierung des Gewebes gewährleistet sein.

Während des Koagulationsvorganges wirken die erste Koagulationselektrode 30a und die zweite Koagulationselektrode 40a als bipolare Anordnung, wobei die Schneidelektrode 50 vorzugsweise elektrisch neutral ist. Während des Schneidvorganges würde die Schneidelektrode 50 hingegen über den Aufnahme-Durchlass-Bereich 60 mit der zweiten Elektrode 40a wechselwirken und eine unkontrollierte Funkenbildung hervorrufen. Zur Vermeidung derartiger unerwünschter Effekte weist die zweite Elektrode 40a daher eine Isolationsschicht 65 an Flächenbereichen auf, die in Richtung der Schneidelektrode 50 ausgebildet sind. Das heißt, dass im Wesentlichen der Aufnahme-Durchlass-Bereich 60 mit der Isolationsschicht 65 ausgekleidet ist. Während des Schneidens bilden dann die erste Elektrode 30a und die Schneidelektrode 50 die bipolare Anordnung aus.

Fig. 2 zeigt, dass die Schneidelektrode 50 ein schraubenlinienförmig ausgebildetes Stabelement umfasst, der Schneidbereich 51 als Schraubenkurve auf dem Stabelement ausgebildet ist und der Abschnitt des Schneidbereiches 51 entlang der Schraubenkurve an dem Stabelement bei Drehung der Schneidelektrode 50 entlang läuft. Das Stabelement ist dafür vorgesehen, sich um die Rotationsachse 52 zu drehen. Dabei kann das Stabelement derart gewickelt sein, dass der Abschnitt des Schneidbereiches 51 entlang einer Fläche des Schneidbereiches verläuft oder aber entlang einer Kante. Verläuft der Abschnitt entlang der Kante, so ist an diesem eine höhere Stromdichte erzielbar.

Fig. 3 zeigt die Fasselement- und/oder Elektrodenanordnung gesäß Fig. 2 in einer Vorderansicht im Schnitt, wobei das zu behandelnde Gewebe 80 zwischen der ersten Koagulationselektrode 30a und der zweiten Koagulationselektrode 40a eingeklemmt ist. Mittels Schneidfunken 70 wird das eingeklemmte Gewebe 80 durchtrennt.

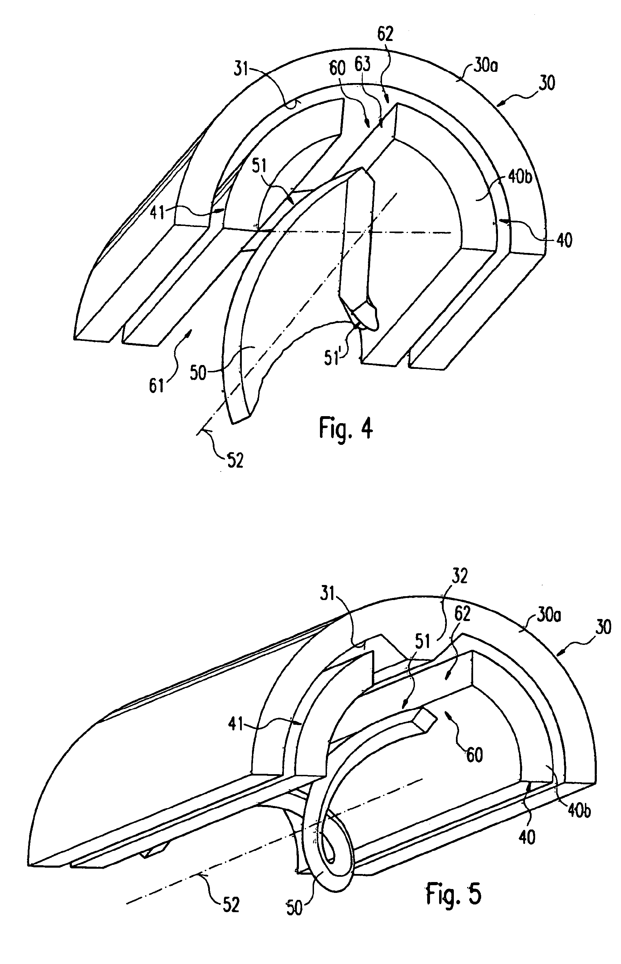

Eine zweite bevorzugte Ausführungsform der erfindungsgemäßen Fasselement- und/oder Elektrodenanordnung ist in Fig. 4 gezeigt. Die Fasselemente 30, 40 sind hier ebenfalls gekrümmt ausgebildet, so dass das zu behandelnde Gewebe zwischen den Fasselementen 30, 40 gespannt werden kann. Fig. 4 zeigt, dass die Schneidelektrode 50 ein tordiertes Flächenelement umfasst, an dessen Rändern zwei Schneidbereiche 51, 51' als Schraubenkurven ausgebildet sind und der Abschnitt des jeweiligen Schneidbereiches 51, 51' entlang der Schraubenkurven an dem Flächenelement bei Drehung der Schneidelektrode entlang läuft. Die so ausgebildete Schneidelektrode 50 ist äußerst stabil und widersteht daher in hohem Maße mechanischen Beanspruchungen. Eine explizite Isolationsschicht an den in Richtung der Schneidelektrode 50 weisenden Flächenbereichen des zweiten Fasselementes 40 bzw. an dem Aufnahme-Durchlass-Bereich 60 zur Vermeidung einer Wechselwirkung zwischen Schneidelektrode 50 und zweitem Fasselement 40 ist in diesem Falle nicht vorgesehen, weil das Fasselement 40 z. B. als elektrisch isolierendes Fasselement 40b ausgebildet ist. Die Wechselwirkung ist somit ausgeschlossen. Die Schneidelektrode 50 und die erste Elektrode 30a bilden in dieser Ausführungsform zur Durchführung des Schneidvorganges eine bipolare Anordnung aus.

Das Fasselement 40 könnte alternativ als Elektrode vorgesehen sein, so dass der Aufnahme-Durchlass-Bereich mit der Isolationsschicht ausgekleidet sein müsste.

Außerdem ist es möglich und wie bereits unter Fig. 1 beschrieben, dass beide Fasselemente 30, 40 aus elektrisch isolierendem Material ausgebildet sind, so dass die Schneidelektrode als einzige Elektrode an dem elektrochirurgischen Instrument vorgesehen ist. Die an dem Patienten angebrachte Neutralelektrode würde dann zusammen mit der Schneidelektrode eine monopolare Anordnung ausbilden.

Fig. 5 zeigt eine perspektivisch dargestellte Fasselement- und/oder Elektrodenanordnung gemäß einer dritten bevorzugten Ausführungsform. Die Anordnung entspricht im Wesentlichen den in den Fig. 2 bis 4 gezeigten Anordnungen und lässt sich ebenfalls gemäß oben beschriebener Varianten ausbilden. Bei diesem Ausführungsbeispiel weist jedoch das dem Aufnahme-Durchlass-Bereich 60 gegenüberliegende Fasselement 40 eine hervorstehenden Bereich 32 in Richtung Aufnahme-Durchlass-Bereich 60 zur Heranführung des zu behandelnden Gewebes an und/oder zur Einführung des zu behandelnden Gewebes in den Aufnahme-Durchlass-Bereich 60 auf. Damit lässt sich die Schnittführung präzisieren, weil z. B. der Schneidfunken vorzugsweise nur innerhalb des Durchlassbereiches 62 auftritt. Zudem bleibt eine Feuchtigkeit des Gewebes im Durchlassbereich 62 länger erhalten, so dass sich eine notwendige Schnittenergie reduzieren lässt.

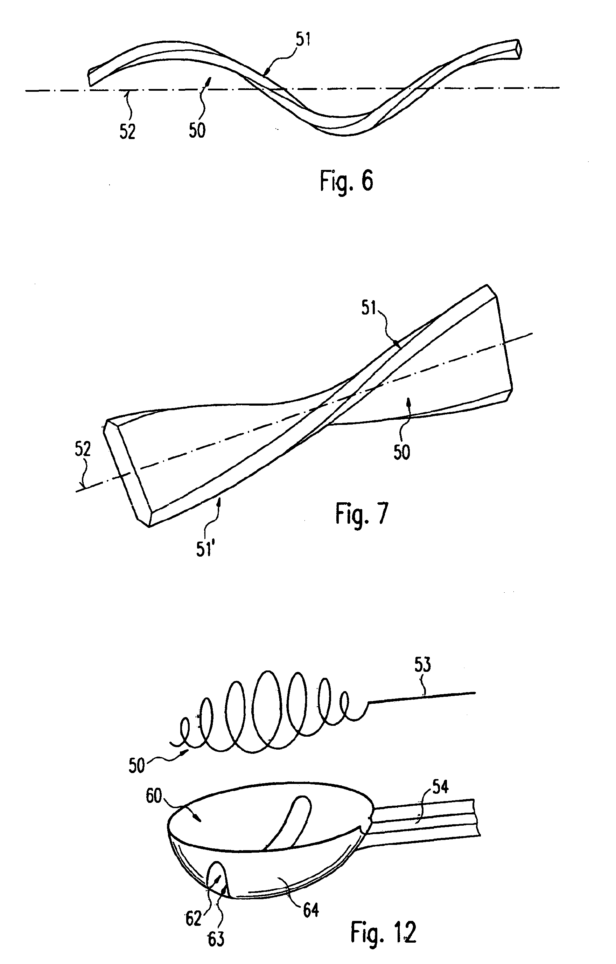

Die Fig. 6 und 7 zeigen jeweils einzeln dargestellte Schneidelektroden 50, wie sie bereits mit den Fig. 2 bis 5 beschrieben wurden.

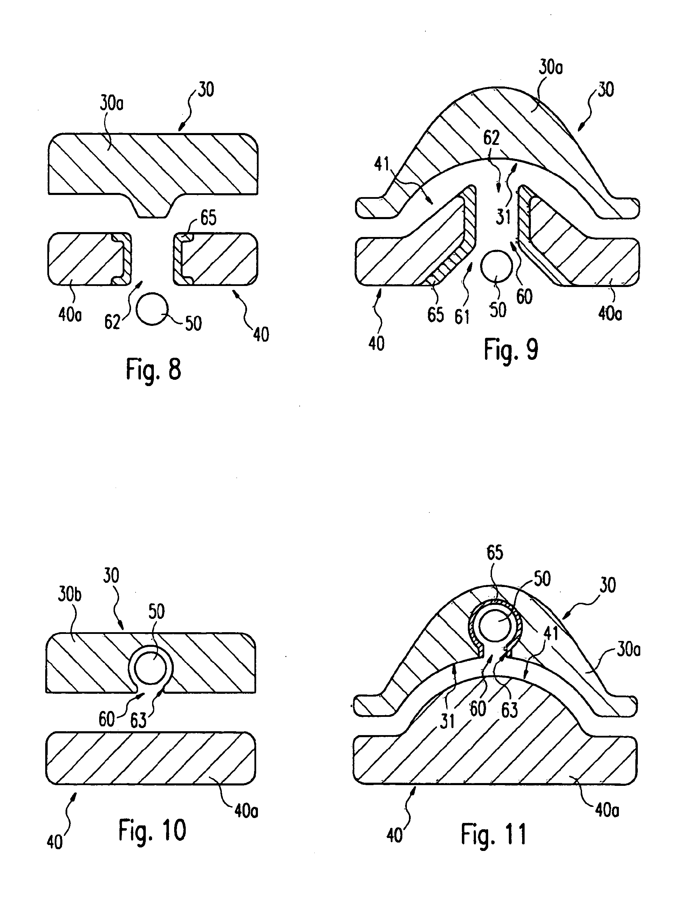

Weitere Fasselement- und/oder Elektrodenanordnungen sind in Fig. 8 mit einer vierten bevorzugten Ausführungsform, in Fig. 9 mit einer fünften bevorzugten Ausführungsform, in Fig. 10 mit einer sechsten bevorzugten Ausführungsform und in Fig. 11 mit einer siebten bevorzugten Ausführungsform dargestellt. Die Zeichnungen zeigen die Fasselement- und/oder Elektrodenanordnungen von vorne, jedoch schematisch im Schnitt. Es sei erwähnt, dass die in den Fig. gezeigten Fasselemente, wie bereits oben beschrieben, jeweils als Koagulationselektroden und/oder als elektrisch isolierende Elemente ausgebildet sein können.

Fig. 8 zeigt in Bezug auf die äußere Gestalt die Fasselemente 30, 40 mit im Wesentlichen rechteckförmigem Querschnitt. Die Fasselemente sind beispielsweise als erste Koagulationselektrode 30a und als zweite Koagulationselektrode 40a ausgebildet. Die zweite Elektrode 40a weist den Aufnahme-Durchlass-Bereich 60 für die Schneidelektrode 50 auf, wobei der Aufnahme-Durchlass-Bereich 60 in diesem Falle im Wesentlichen aus dem Durchlassbereich 62 besteht. Die Schneidelektrode 50, hier als Kreis angedeutet, ist demgemäß unterhalb der zweiten Elektrode 40a gelagert. Um die Wechselwirkung zwischen der Schneidelektrode 50 und der zweiten Elektrode 40a zu vermeiden, weist die zweite Elektrode 40a an dem Aufnahme-Durchlass-Bereich 60 an den Flächenbereichen, die im Wesentlichen der Schneidelektrode 50 zugewandt sind, die Isolationsschicht 65 auf. Damit bilden die Schneidelektrode 50 und die erste Elektrode 30a während des Schneidvorganges die bipolare Anordnung aus. Die erste Elektrode 30a weist den hervorstehenden Bereich 32 in Richtung Aufnahme-Durchlass-Bereich 60 zur Heranführung des zu behandelnden Gewebes an und/oder zur Einführung des zu behandelnden Gewebes in den Aufnahme-Durchlass-Bereich 60 auf.

Die Anordnung der Fasselemente 30, 40 und der Schneidelektrode 50 gemäß Fig. 9 entspricht im Wesentlichen derjenigen aus Fig. 8. Auch hier sind die Fasselement als erste Koagulationselektrode 30a und als zweite Koagulationselektrode 40a ausgebildet, weisen jedoch eine gekrümmte Gestalt und daher die oben beschriebenen Spannbereiche 31, 41 zum Fixieren und Straffen des zu behandelnden Gewebes auf. Der Aufnahme-Durchlass-Bereich 60 ist in Richtung einer von Fassflächen der Fasselemente 30, 40 abgewandten Seite trichterförmig ausgebildet, so dass die Schneidelektrode 50 geschützt innerhalb der zweiten Elektrode untergebracht ist.

Fig. 10 zeigt wiederum Fasselemente 30, 40, die im Wesentlichen einen rechteckförmigen Querschnitt aufweisen. Da die Schneidelektrode 50 hier zwischen dem ersten Fasselement 30 und dem zweiten Fasselement 40 ausgebildet ist, befindet sich der Aufnahme-Durchlass-Bereich 60 hier beispielsweise innerhalb des ersten Fasselements 30. In diesem Falle ist das erste Fasselement als elektrisch isolierendes Element 30b und das zweite Fasselement als Koagulationselektrode 40a ausgebildet, so dass die Schneidelektrode 50 mit der zweiten Elektrode 40a die bipolare Anordnung ausbildet.

Fig. 11 entspricht im Wesentlichen Fig. 10, wobei die Fasselemente hier als erste Koagulationselektrode 30a und als zweite Koagulationselektrode 40a ausgebildet sind und die Spannbereiche 31, 41 aufweisen. Der Aufnahme-Durchlass-Bereich 60 ist innerhalb der ersten Elektrode 30a angeordnet, so dass dieser mit der Isolationsschicht 65 ausgekleidet ist. Damit wird die Wechselwirkung der Schneidelektrode 50 mit der ersten Elektrode 30a während des Schneidvorganges vermieden.

Unabhängig von der Ausgestaltung der Fasselemente kann die Schneidelektrode 50 jeweils selbst als Schraubenkurve oder dergleichen um die Rotationsachse 52 verlaufend Raumkurve ausgebildet sein, also z. B. als gewickeltes Stabelement oder als tordiertes Blech oder aber das gewickelte Stabelement beispielsweise umfassen.

Die in den Fig. gezeigten Aufnahme-Durchlass-Bereiche 60 sind vorzugsweise von ihren Ausmaßen derart ausgestaltet, dass sie den definierten Fensterbereich 63 ausbilden. Damit wird stets sichergestellt, dass nur ein definierter Gewebebereich für den Schneidvorgang zugänglich ist.

Es lassen sich auch andere Formgebungen für die Schneidelektrode und den Aufnahme-Durchlass-Bereich vorsehen. Insbesondere kann der Aufnahme-Durchlass-Bereich an bzw. in einem zusätzlichen, ggf. auswechselbaren Element an dem elektrochirurgischen Instrument ausgebildet sein, d. h. eine elektrochirurgische Anordnung weist neben den Elektroden ein explizites Element für den Aufnahme-Durchlass-Bereich auf (so ließe sich z. B. ein vorgegebener Fensterbereich eines Fasselements variieren). Das Element 64 kann aber auch lediglich als gekrümmtes Fasselement ausgebildet sein, so wie es mit obigen Ausführungsbeispielen bereits beschrieben wurde. So ist in Fig. 12 schematisch eine gewickelte Schneidelektrode 50 dargestellt, die im Wesentlichen die Ummantelung eines Rotationsellipsoids beschreibt. Diese Elektrode 50 ist beispielsweise in dem entsprechend muldenförmigen Element 64 für den Aufnahme-Durchlass-Bereich 60 anzuordnen, wobei das Element 64 z. B. als elektrisch isolierendes Element oder selbst als Elektrode ausgebildet ist. Als Gegenelektrode kann, je nach Ausführungsform, eine Neutralelektrode oder eine über das muldenförmige Element 64 stülpbare weitere Elektrode (nicht gezeigt) vorgesehen sein, so dass das zu behandelnde Gewebe zwischen dem Element 64 und der weiteren Elektrode eingeklemmt ist. Der Durchlassbereich 62 ist vorzugsweise als der definierte Fensterbereich 63 ausgebildet. Aufgrund des muldenförmigen Elements 64 ist der Fensterbereich 63 derart gekrümmt ausgebildet, dass sich beispielsweise ein präziser Schnitt entlang des gekrümmten Fensterbereichs durchführen lässt. Über den Rotationsantrieb und eine in einer Führung 54 des Elements gelagerte Antriebswelle 53 wird die Schneidelektrode 50 in Rotation versetzt.

An dieser Stelle sei darauf hingewiesen, dass alle oben beschriebenen Teile für sich alleine gesehen und in jeder Kombination, insbesondere die in den Zeichnungen dargestellte Details als erfindungswesentlich beansprucht werden. Abänderungen hiervon sind dem Fachmann geläufig.

### Bezugszeichenliste

- 10: Elektrochirurgisches Instrument
- 11: Distales Ende
- 12: Distales Ende
- 13: Proximales Ende
- 14: Proximales Ende
- 15: Branche
- 16: Branche
- 17: Achse
- 18: Griffteil
- 19: Griffteil
- 20: Stromanschlusselement, Stromzuführungseinrichtung
- 21: Rotationsantrieb
- 21': Fingerrad
- 30: Erstes Fasselement
- 30a: Erste Koagulationselektrode
- 30b: Elektrisch isolierendes Element
- 31: Spannbereich
- 32: Hervorstehender Bereich
- 40: Zweites Fasselement
- 40a: Zweite Koagulationselektrode
- 40b: Elektrisch isolierendes Element
- 41: Spannbereich
- 50: Schneidelektrode
- 51: Schneidbereich
- 51': Schneidbereich
- 52: Rotationsachse
- 53: Antriebswelle
- 54: Führung
- 60: Aufnahme-Durchlass-Bereich
- 61: Aufnahmebereich
- 62: Durchlassbereich
- 63: Definierter Fensterbereich
- 64: Element für den Aufnahme-Durchlass-Bereich
- 65: Isolationsschicht
- 70: Lichtbogen, Schneidfunke
- 80: Gewebe

## Patentansprüche

1. Elektrochirurgisches Instrument zum Schneiden und/oder Koagulieren eines zu behandelnden Gewebes (80) mit
- mindestens einer Schneidelektrode (50) zum Durchleiten eines HF-Stromes durch das Gewebe (80) zum Ausführen eines Schneidvorganges,
- Stromzuführungseinrichtungen (20) zum Zuführen des HF-Stromes mindestens zu der Schneidelektrode (50), und
- einen Aufnahme Durchlass Bereich (60) mit einem Aufnahmebereich (61),
und einen definierten Fensterbereich (63), wobei die Schneidelektrode (50) in dem Aufnahmebereich (61) aufgenommen ist, und wobei ein Lichtbogen über den Fensterbereich (63) zwischen der Schneidelektrode (50) und dem Gewebe (80) erzeugbar ist,
**dadurch gekennzeichnet, dass**
die Schneidelektrode (50) um eine Rotationsachse (52) retativ zu dem Fensterbereich (63) drehbar an dem elektrochirurgischen Instrument (10) angeordnet ist,
wobei die Schneidelektrode (50) mindestens einen Schneidbereich (51) aufweist, der derart als Schraubenkurve um die Rotationsachse (52) verlaufende Raumkurve ausgebildet und angeordnet ist, dass er bei Drehung der Schneidelektrode (50) um die Rotationsachse (52) über seine Länge hinweg jeweils dann abschnittsweise an dem Gewebe (80) wirksam wird, wenn dieser Abschnitt einen definierten Mindestabstand zu dem Gewebe (80) unterschreitet und innerhalb des Fensterbereiches (63) liegt.

2. Elektrochirurgisches Instrument nach Anspruch 1,
wobei die Schneidelektrode (50) ein schraubenlinienförmig ausgebildetes Stabelement umfasst, der Schneidbereich (51) als Schraubenkurve auf dem Stabelement ausgebildet ist und der Abschnitt des Schneidbereiches (51) entlang der Schraubenkurve an dem Stabelement bei Drehung der Schneidelektrode entlang läuft.

3. Elektrochirurgisches Instrument nach Anspruch 1 oder 2, insbesondere nach Anspruch 1,
wobei die Schneidelektrode (50) ein tordiertes Flächenelement umfasst, an dessen Rändern zwei Schneidbereiche (51, 51') als Schraubenkurven ausgebildet sind und der Abschnitt der Schneidbereiche (51, 51') entlang der Schraubenkurven an dem Flächenelement bei Drehung der Schneidelektrode entlang läuft.

4. Elektrochirurgisches Instrument nach einem der vorhergehenden Ansprüche,
wobei die Schneidelektrode (50) mit mindestens einer Windung ausgebildet ist.

5. Elektrochirurgisches Instrument nach einem der vorhergehenden Ansprüche,
wobei zwei gelenkig miteinander verbundene Branchen (15, 16) vorgesehen sind, die entsprechend einem Klemm- oder Schneidwerkzeug betätigbar sind und somit eine Halteeinrichtung mit jeweils mindestens einem Fasselement (30, 40) an den Branchen (15, 16) zum Fassen des zu behandelnden Gewebes (80) ausbilden,
wobei mindestens eines der Fasselemente (30, 40) den Aufnahme Durchlass Bereich (60) mit einem Aufnahmebereich (61) und den Fensterbereich (63) derart aufweist,
daß die Schneidelektrode (50) in oder an den Fasselementen (30, 40) in dem Aufnahmebereich (61) lagerbar und ein Lichtbogen über den Fensterbereich (63) zwischen der Schneidelektrode und dem in der Halteeinrichtung eingeklemmten Gewebe (80) erzeugbar ist.

6. Elektrochirurgisches Instrument nach einem der vorhergehenden Ansprüche, insbesondere nach Anspruch 5,
wobei die Halteeinrichtung eine erste Elektrode (30a) und eine zweite Elektrode (40a) zum Durchleiten eines Koagulationsstromes für den Koagulationsvorgang durch das Gewebe (80) als Fasselemente an jeweils den Branchen (15, 16) aufweist.

7. Elektrochirurgisches Instrument nach einem der vorhergehenden Ansprüche, insbesondere nach Anspruch 6,
wobei der Aufnahme-Durchlass-Bereich (60) mindestens an in Richtung der Schneidelektrode (50) weisenden Flächenbereichen eine Isolationsschicht (64) aufweist, so dass die Elektrode, die der Elektrode mit dem Aufnahme-Durchlass-Bereich (60) gegenüberliegt und die Schneidelektrode (50) während des Schneidvorganges eine bipolare Anordnung ausbilden.

8. Elektrochirurgisches Instrument nach einem der vorhergehenden Ansprüche, insbesondere nach Anspruch 5,
wobei die Halteeinrichtung eine Elektrode (30a, 40a) und ein elektrisch isolierendes Fasselement (30b, 40b) aufweist, wobei die Elektrode (30a, 40a) und die Schneidelektrode (50) derart zueinander angeordnet sind, dass sie während des Schneidvorganges eine bipolare Anordnung ausbilden.

9. Elektrochirurgisches Instrument nach einem der vorhergehenden Ansprüche, insbesondere nach einem der Ansprüche 5 bis 8,
wobei das dem Aufnahme-Durchlass-Bereich (60) gegenüberliegende Fasselement (30, 40) einen hervorstehenden Bereich (32) in Richtung Aufnahme-Durchlass-Bereich (60) zur Heranführung des zu behandelnden Gewebes (80) an und/oder zur Einführung des zu behandelnden Gewebes (80) in den Aufnahme-Durchlass-Bereich (60) aufweist.

10. Elektrochirurgisches Instrument nach einem der vorhergehenden Ansprüche, insbesondere nach einem der Ansprüche 5 bis 9,
wobei die Fasselemente (30, 40) jeweils mindestens einen Spannbereich (31, 41) aufweisen, derart, dass beim Einklemmen des Gewebes (80) dieses zwischen den Fasselementen (30, 40) vorgespannt ist und der Schneidvorgang mittels der Schneidelektrode (50) an dem vorgespannten Gewebe (80) durchführbar ist.

11. Elektrochirurgisches Instrument nach einem der vorhergehenden Ansprüche, insbesondere nach Anspruch 10,
wobei einer der Spannbereiche (41) mindestens in einem ersten mittleren Abschnitt konvex, der ihm gegenüberliegende Spannbereich (31) mindestens in einem zweiten mittleren Abschnitt konkav gekrümmt ist, so dass bei einem Zusammenführen der Branchen (15, 16) die Spannbereiche (31, 41) im Wesentlichen formschlüssig ineinander passen.

12. Elektrochirurgisches Instrument nach einem der vorhergehenden Ansprüche,
wobei dem elektrochirurgischen Instrument (10) ein Rotationsantrieb (21) zur Drehung der Schneidelektrode (50) zugeordnet ist.

## Claims

1. Electrosurgical instrument for cutting and/or coagulation of tissue (80) to be treated, comprising
- at least one cutting electrode (50) for passing an HF current through the tissue (80) for performing a cutting procedure,
- current supply arrangements (20) for supplying the HF current at least to the cutting electrode (50), and
- an accommodating/let-through region (60) having an accommodating region (61) and a defined window region (63), the cutting electrode (50) being accommodated in the accommodating region (61) and an electric arc being producible between the cutting electrode (50) and the tissue (80) by way of the window region (63),
**characterised in that**
the cutting electrode (50) is arranged on the electrosurgical instrument (10) so as to be rotatable about a rotation axis (52) relative to the window region (63),
the cutting electrode (50) having at least one cutting region (51), which is so provided and arranged as a 3-dimensional curve running around the rotation axis (52) in the form of a helix that, on rotation of the cutting electrode (50) about the rotation axis (52), that region comes, portion-by-portion over its length, to act upon the tissue (80) in each case when said portion is located at less than a defined minimum spacing from the tissue (80) and lies within the window region (63).

2. Electrosurgical instrument according to claim 1,
wherein the cutting electrode (50) comprises a bar element provided in helically linear form, the cutting region (51) is provided in the form of a helix on the bar element, and the portion of the cutting region (51) along the helix runs along the bar element on rotation of the cutting electrode.

3. Electrosurgical instrument according to claim 1 or 2, especially according to claim 1, wherein the cutting electrode (50) comprises a twisted planar element, at the rims of which two cutting regions (51, 51') are provided in the form of helices, and the portion of the cutting regions (51, 51') along the helices runs along the planar element on rotation of the cutting electrode.

4. Electrosurgical instrument according to one of the preceding claims, wherein the cutting electrode (50) is provided in a form having at least one turn.

5. Electrosurgical instrument according to one of the preceding claims, wherein there are provided two limbs (15, 16) in articulated connection with one another, which are actuatable in a manner corresponding to a clamping or cutting tool and which accordingly form a holding device having at least one grasping element (30, 40) on each of the limbs (15, 16) for grasping the tissue (80) to be treated,
wherein at least one of the grasping elements (30, 40) has the accommodating/let-through region (60) having an accommodating region (61) and the window region (63)
so that the cutting electrode (50) can be housed in the accommodating region (61) in or on the grasping elements (30, 40) and an electric arc can be produced between the cutting electrode and the tissue (80) clamped in the holding device, by way of the window region (63).

6. Electrosurgical instrument according to one of the preceding claims, especially according to claim 5,
wherein the holding device has a first electrode (30a) and a second electrode (40a) for passing a coagulation current for the coagulation procedure through the tissue (80), in the form of grasping elements on each of the limbs (15, 16).

7. Electrosurgical instrument according to one of the preceding claims, especially according to claim 6,
wherein the accommodating/let-through region (60) has, at least on surface regions facing in the direction of the cutting electrode (50), an insulating layer (64) so that the electrode located opposite the electrode with the accommodating/let-through region (60) and the cutting electrode (50) form a bipolar arrangement during the cutting procedure.

8. Electrosurgical instrument according to one of the preceding claims, especially according to claim 5,
wherein the holding device has an electrode (30a, 40a) and an electrically insulating grasping element (30b, 40b), the electrode (30a, 40a) and the cutting electrode (50) being so arranged in relation to one another that they form a bipolar arrangement during the cutting procedure.

9. Electrosurgical instrument according to one of the preceding claims, especially according to one of claims 5 to 8,
wherein the grasping element (30, 40) located opposite the accommodating/let-through region (60) has a projecting region (32) in the direction of the accommodating/let-through region (60) for bringing the tissue (80) to be treated close to, and/or for introducing the tissue (80) to be treated into, the accommodating/let-through region (60).

10. Electrosurgical instrument according to one of the preceding claims, especially according to one of claims 5 to 9,
wherein each of the grasping elements (30, 40) has at least one tensioning region (31, 41) so that when the tissue (80) is clamped in place it is pre-tensioned between the grasping elements (30, 40) and the cutting procedure can be performed on the pre-tensioned tissue (80) by means of the cutting electrode (50).

11. Electrosurgical instrument according to one of the preceding claims, especially according to claim 10,
wherein one of the tensioning regions (41) is convexly curved at least in a first central portion and the tensioning region (31) located opposite it is concavely curved at least in a second central portion so that, when the limbs (15, 16) are brought together, the tensioning regions (31, 41) substantially fit one into the other interengagingly.

12. Electrosurgical instrument according to one of the preceding claims,
wherein a rotary drive (21) for rotation of the cutting electrode (50) is associated with the electrosurgical instrument (10).

## Revendications

1. Instrument électrochirurgical de section et/ou coagulation d'un tissu à traiter (80) avec
- au moins une électrode de coupe (50) pour faire passer un courant HF à travers le tissu (80) en vue d'effectuer une incision,
- des dispositifs d'alimentation en courant (20) pour amener le courant HF au moins jusqu'à l'électrode de coupe (50), et
- un logement (61) et une fenêtre définie (63),
l'électrode de coupe (50) étant agencée dans le logement (61) et un arc électrique pouvant être produit à travers la fenêtre (63) entre l'électrode de coupe (50) et le tissu (80),
**caractérisé en ce que**
l'électrode de coupe (50) est montée sur l'instrument électrochirurgical (10) en étant mobile en rotation autour d'un axe de rotation (52) par rapport à la fenêtre (63),
l'électrode de coupe (50) présentant au moins une zone de coupe (51) conçue et agencée selon une courbe en trois dimensions s'enroulant de manière hélicoïdale autour de l'axe de rotation (52), de telle sorte que la zone de coupe devient active sur le tissu (80), au niveau d'une portion de sa longueur, lors de la rotation de l'électrode de coupe (50) autour de l'axe de rotation (52), lorsque ladite portion est éloignée du tissu (80) d'une distance inférieure à une distance minimale définie et se trouve au niveau de la fenêtre (63).

2. Instrument électrochirurgical selon la revendication 1,
dans lequel l'électrode de coupe (50) comprend une tige de forme hélicoïdale, la zone de coupe (51) étant prévue sur la tige sous la forme d'une courbe hélicoïdale et la portion de la zone de coupe (51) se déplaçant sur la tige, le long de la courbe hélicoïdale, lors de la rotation de l'électrode.

3. Instrument électrochirurgical selon la revendication 1 ou la revendication 2, notamment selon la revendication 1,
dans lequel l'électrode de coupe (50) comprend un élément de surface torsadé sur les bords duquel deux zones de coupe (51, 51') décrivent des courbes hélicoïdales, la portion des zones de coupe (51, 51') se déplaçant sur l'élément de surface, le long des courbes hélicoïdales, lors de la rotation de l'électrode de coupe.

4. Instrument électrochirurgical selon l'une quelconque des revendications précédentes,
dans lequel l'électrode de coupe (50) comprend au moins une spire.

5. Instrument électrochirurgical selon l'une quelconque des revendications précédentes,
dans lequel il est prévu deux branches (15, 16) reliées entre elles de manière articulée, actionnables à la manière d'une pince ou d'un instrument de coupe et formant ainsi un dispositif de maintien avec au moins un élément de préhension (30, 40) sur chacune des branches (15, 16) pour saisir le tissu à traiter (80),
au moins un des éléments de préhension (30, 40) présentant le logement (61) et la fenêtre (63) de telle sorte que l'électrode de coupe (50) peut être montée dans le logement (61) des éléments de préhension (30, 40) et qu'un arc électrique peut être produit à travers la fenêtre (63), entre l'électrode de coupe et le tissu (80) pincé dans le dispositif de maintien.

6. Instrument électrochirurgical selon l'une quelconque des revendications précédentes, notamment selon la revendication 5,
dans lequel le dispositif de maintien présente sur les branches (15, 16), en tant qu'éléments de préhension, une première électrode (30a) et une deuxième électrode (40a) destinées à faire passer un courant de coagulation à travers le tissu (80) dans le cadre du processus de coagulation.

7. Instrument électrochirurgical selon l'une quelconque des revendications précédentes, en particulier selon la revendication 6,
dans lequel la zone de logement/passage (60) présente une couche d'isolation (64) au moins sur des éléments de surface orientés en direction de l'électrode de coupe (50), de sorte que l'électrode en regard de l'électrode présentant la zone de logement/passage (60) et l'électrode de coupe (50) forment un agencement bipolaire pendant l'incision.

8. Instrument électrochirurgical selon l'une quelconque des revendications précédentes, en particulier selon la revendication 5,
dans lequel le dispositif de maintien comprend une électrode (30a, 40a) et un élément de préhension électriquement isolant (30b, 40b), l'électrode (30a, 40a) et l'électrode de coupe (50) étant agencées l'une par rapport à l'autre de manière à former un agencement bipolaire pendant l'incision.

9. Instrument électrochirurgical selon l'une quelconque des revendications précédentes, en particulier selon l'une des revendications 5 à 8,
dans lequel l'élément de préhension (30, 40) en regard de la zone de logement/passage (60) présente une zone en saillie (32) en direction de la zone de logement/passage (60) pour approcher le tissu à traiter (80) de la zone de logement/passage (60) et/ou introduire le tissu à traiter (80) dans la zone de logement/passage (60).

10. Instrument électrochirurgical selon l'une quelconque des revendications précédentes, en particulier selon l'une quelconque des revendications 5 à 9,
dans lequel les éléments de préhension (30, 40) présentent respectivement au moins une zone de mise en tension (31, 41), de telle sorte que lors du pincement du tissu (80), ce dernier est prétendu entre les éléments de préhension (30, 40) et que l'incision peut être effectuée au moyen de l'électrode de coupe (50) sur le tissu prétendu (80).

11. Instrument électrochirurgical selon l'une quelconque des revendications précédentes, en particulier selon la revendication 10,
dans lequel une des zones de mise en tension (41) est incurvée de manière convexe sur au moins une première portion médiane, la zone de serrage (31) en regard étant incurvée de manière concave sur au moins une deuxième portion médiane, de sorte que lorsque les branches (15, 16) sont rapprochées l'une de l'autre, les zones de mise en tension (31, 41) s'engagent l'une dans l'autre sensiblement par engagement positif.

12. Instrument électrochirurgical selon l'une quelconque des revendications précédentes,
dans lequel un dispositif d'entraînement en rotation (21) est associé à l'instrument électrochirurgical (10) pour entraîner en rotation l'électrode de coupe (50).
